Europäisches Patentamt

European Patent Office

Office européen des brevets

(19)

(11) **EP 0 835 100 B1**

(12) **EUROPÄISCHE PATENTSCHRIFT**

(45) Veröffentlichungstag und Bekanntmachung des Hinweises auf die Patenterteilung:
**02.01.2002 Patentblatt 2002/01**

(51) Int Cl.⁷: **A61K 9/127**, A61K 31/52

(21) Anmeldenummer: **96919764.9**

(86) Internationale Anmeldenummer:
**PCT/EP96/02012**

(22) Anmeldetag: **10.05.1996**

(87) Internationale Veröffentlichungsnummer:
**WO 96/35412 (14.11.1996 Gazette 1996/50)**

(54) **TOPISCHE ACICLOVIR-ZUBEREITUNG**

TOPICAL ACICLOVIR PREPARATION

PREPARATION TOPIQUE D'ACICLOVIR

(84) Benannte Vertragsstaaten:
**AT BE CH DE DK ES FI FR GB GR IE IT LI LU NL PT SE**
Benannte Erstreckungsstaaten:
**SI**

(30) Priorität: **10.05.1995 DE 19517147**

(43) Veröffentlichungstag der Anmeldung:
**15.04.1998 Patentblatt 1998/16**

(73) Patentinhaber: **HEXAL AG**
**D-83607 Holzkirchen (DE)**

(72) Erfinder:
• FISCHER, Wilfried
D-83607 Holzkirchen (DE)
• BRACHER, Daniel
D-83607 Holzkirchen (DE)

(74) Vertreter: **Boeters, Hans Dietrich, Dr. et al**
**Patentanwälte Boeters & Bauer, Bereiteranger 15**
**81541 München (DE)**

(56) Entgegenhaltungen:
EP-A- 0 523 418        EP-A- 0 582 239
WO-A-95/03805        WO-A-95/35095
IT-A- 1 249 472

**Beschreibung**

Aciclovir (9-[2-Hydroxyethoxy-methyl]-guanin) und seine pharmazeutisch verträglichen Derivate, wie beispielsweise Salze und Ester, sind in vivo und in vitro wirksam gegen DNA-Viren, beispielsweise gegen Cytomegalo-, Adeno-, Rhino-, Mengo-, Sindbis- und infektiöse Mononucleosis-Viren und insbesondere gegen Vaccinia- und Herpes-Viren; vergleiche beispielsweise DE-A-2 539 963. Im Stand der Technik finden sich diverse Vorschläge für eine topische Applikation der Wirkstoffe. So sieht WO-A-9 415 614 eine Dispersion des Wirkstoffs in einem wäßrigen Gel (Träger) mit einem Carbonsäuresalz vor. Nach EP-A-0 394 928 wird der Wirkstoff in einer Creme mit einem Exzipienten und einem nicht-ionischen oberflächenaktiven Mittel vorgesehen. DE-A-3 521 143 schlägt den Wirkstoff in einem fett-freien Gel vor. Nach EP-A-0 137 807 und EP-A-0 145 779 wird der Wirkstoff in einem Lösungsmittel (Träger) mit oder aus Dimethylsulfoxid appliziert.

Eine Schwierigkeit bei der Applikation von Aciclovir besteht darin, daß es eine geringe Wasserlöslichkeit besitzt und in hydrophoben Lösungsmitteln nahezu unlöslich ist; vgl. beispielsweise EP-B-0 044 543, wo eine Zusammensetzung mit Aciclovir als Wirkstoff in einer wässerigen Phase mit einer darin dispergierten Ölphase beschrieben wird, wobei die Gegenwart eines mehrwertigen Alkohols als Co-Solvens die Permeation fördern soll. Analog wird mit WO-A1-9 405 258 eine Zubereitung mit einem Gehalt an Aciclovir vorgeschlagen, bei der es sich wiederum um eine Öl-in-Wasser-Emulsion mit einem Gehalt an einem mehrwertigen Alkohol handelt.

WO-A1-9 420 072 schlägt vor, ein bei Raumtemperatur festes Matrixmaterial, insbesondere Fett (Seite 13 Zeilen 21/22), zu schmelzen, danach in der Schmelze einen Wirkstoff zu lösen (vgl. Seite 43 Zeilen 24/26) und danach die Schmelze in Gegenwart von Wasser abzukühlen und zu verfestigen. Das in einer Liste von Wirkstoffen erwähnte Aciclovir löst sich jedoch nicht in Fetten und Ölen.

Aufgabe der vorliegenden Erfindung ist es, eine Zubereitung mit einem Gehalt an Aciclovir oder einem Aciclovir-Derivat zur topischen Applikation vorzusehen, wobei die Zubereitung eine befriedigende Wirkstoff-Löslichkeit und eine befriedigende Lagerbeständigkeit bieten soll.

Diese Aufgabe wird nun erfindungsgemäß durch eine wäßrige pharmazeutische Zubereitung zu topischer Applikation mit einem Gehalt an Aciclovir oder einem Aciclovir-Derivat als Wirkstoff, einem mehrwertigen Alkohol, einer öligen Komponente und einem fakultativen Konsistenzgeber gelöst, wobei die Zubereitung durch einen Gehalt an einem Phospholipid gekennzeichnet ist.

Für pharmazeutisch verträgliche Aciclovir-Derivate kann auf den Stand der Technik verwiesen werden.

Erfindungsgemäß kann man ein ungesättigtes oder ein gesättigtes Lecithin als Phospholipid verwenden, beispielsweise ein hydriertes Lecithin. Bei einer bevorzugten Ausführungsform entspricht der Phospholipid-Gehalt einer Doppel- oder Mehrschichtstruktur des Phospholipids in der wäßrigen pharmazeutischen Zubereitung. Diese Doppeloder Mehrschichtstruktur liegt bei Öl-in-Wasser-Emulsionen des Stands der Technik, beispielsweise gemäß WO-A1-9 405 258 oder EP-A1-0 044 543, nicht vor. So kann der Phospholipid-Gehalt beispielsweise etwa 2 bis etwa 20 und insbesondere bis etwa 10 Gew.-% auf Basis der Zubereitung betragen.

Der Wirkstoff-Gehalt kann etwa 2 bis etwa 7 Gew.-% auf Basis der Zubereitung betragen.

Der Wassergehalt kann etwa 15 bis 50 und insbesondere etwa 25 bis etwa 45 Gew.-% auf Basis der Zubereitung betragen.

Für den mehrwertigen Alkohol als eine der Komponenten der erfindungsgemäßen Zubereitung kann auf den Stand der Technik für pharmazeutische Zubereitungen zu topischer Applikation von Aciclovir oder Aciclovir-Derivaten verwiesen werden. Beispiele verwendbarer mehrwertiger Alkohole sind Propylenglycol, Butan-1,3-diol oder Glycerin. Der Gehalt an mehrwertigem Alkohol kann etwa 25 bis etwa 45 Gew.-% auf Basis der Zubereitung betragen.

Erfindungsgemäß kann der Verhältnis von Wasser zu mehrwertigem Alkohol etwa 1,5:1 bis etwa 0,75:1 betragen.

Als ölige Komponente werden erfindungsgemäß Öle auf pflanzlicher Basis bevorzugt, beispielsweise Avocadoöl, Erdnußöl, Sonnenblumenöl, Sesamöl und Olivenöl. Alternativ oder zusätzlich können als ölige Komponente mittelkettige Triglyceride verwendet werden. Der Gehalt an öliger Komponente kann etwa 5 bis etwa 40 und insbesondere etwa 10 bis etwa 30 Gew.-% auf Basis der Zubereitung betragen.

Ferner kann die erfindungsgemäße Zubereitung einen zusätzlichen Gehalt an α-Tocopherol oder einem α-Tocopherol-Derivat aufweisen, insbesondere von etwa 0,1 bis etwa 5 und insbesondere von etwa 0,5 bis etwa 5 Gew.-% auf Basis der Zubereitung.

Als fakultative Konsistenzgeber kommen schließlich beispielsweise Glycerinmonostearat, Cetylstearylalkohol, synthetische oder natürliche Wachse, beispielsweise Bienenwachs, Cetylpalmitat, Walrat und Polyacrylsäure, wie Carbopol, in Betracht, beispielsweise in einer Menge von etwa 0,5 bis 20 Gew.-%.

Nachstehend wird die Erfindung durch Beispiele näher erläutert.

**Beispiel 1**

Es wurden die folgenden Komponenten bei etwa 70 bis 80 °C aufgeschmolzen und über einen Spalthomogenisator (externe Umpumpung) emulgiert:

| | |
|---|---:|
| hydriertes Lecithin | 6,0 ± 0,5 % |
| Avocadoöl | 10,0 ± 1,0 % |
| mittelkettige Triglyceride | 10,0 ± 1,0 % |
| Propylenglycol | 30,0 ± 0,5 % |
| α-Tocopherol | 2,5 % |
| Wasser | 41,5 % |
| | 100,0 % |

5 Gewichtsteile feingemahlenes Aciclovir (mittlerer Korngrößendurchmesser etwa 15 bis 20 μm) wurden mit 10 Gewichtsteilen Propylenglycol angerieben. Die anfallende Suspension wurde in einer Prozeßanlage bei Raumtemperatur unter Vakuum in 85 Gewichtsteile der zuvor hergestellten Basiszubereitung eingerührt, so daß man ein Endprodukt mit folgender Zusammensetzung erhielt:

| | |
|---|---:|
| hydriertes Lecithin | 5,1 % |
| Avocadoöl | 8,5 % |
| mittelkettige Triglyceride | 8,5 % |
| α-Tocopherol | 2,1 % |
| Propylenglycol | 35,5 % |
| Wasser | 35,3 % |
| Aciclovir | 5,0 % |
| | 100,0 % |

Dieses Endprodukt weist in der kontinuierlichen Wasserphase Schichtstrukturen auf, die aus Phospholipidschichten mit inkorporiertem Ölanteil aufgebaut sind. Daneben liegen oligo- bzw. multi-lamellar geschichtete Phospholipidvesikel (liposomale Strukturen) vor.

**Beispiel 2**

Es wurde Beispiel 1 mit den Ausnahmen wiederholt, daß für die Basiszubereitung ein normales ungesättigtes Lecithin (statt gehärtetem Lecithin) und als Ölkomponente Sojaöl (statt Avocadoöl/mittelkettige Triglyceride) verwendet wurde.

**Beispiel 3 und Vergleichsbeispiel 1**

**Beispiel 3**

Zusammensetzung:

| | |
|---|---:|
| Aciclovir | 5,0 % |
| hydriertes Lecithin | 8,5 % |
| Sonnenblumenöl | 8,3 % |
| α-Tocopherol | 0,5 % |
| Propylenglycol | 33,7 % |
| Wasser | 28,0 % |
| Glycerolmonostearat | 8,0 % |
| mikrokristallines Wachs | 8,0 % |
| | 100,0 % |

Das Wachs und Glycerolmonostearat werden bei 70 bis 80 °C aufgeschmolzen und mit Sonnenblumenöl und Tocopherol versetzt. Anschließend wird das Wasser und ein Teil des Propylenglycols zugegeben und unter Rühren emulgiert. 5 Gewichtsteile Aciclovir werden mit dem Rest Propylenglycol angerieben und bei 50 bis 60 °C zugegeben und durch Rühren homogenisiert.

**Vergleichsbeispiel (Öl-in-Wasser-Emulsion)**

Zusammensetzung:

| | |
|---|---:|
| Aciclovir | 5,0 % |
| Propylenglycol | 40,0 % |
| Wasser | 29,0 % |
| Na-Laurylsulfat | 0,75 % |
| PEG/PPG-Blockcopolymer | 1,0 % |
| Cetylstearylalkohol | 6,75 % |
| Paraffin dickflüssig | 5,0 % |
| Vaseline | 12,5 % |
| | 100,00 % |

Die Herstellung erfolgte gemäß EP-A1-0 044 543 Beispiel 1.

**Bestimmung der in-vitro Hautpermeation**

Prüfbedingungen:

Modifizierte Franz-Zelle, Mäusehaut, Akzeptor 0,9 % NaCl und 0,5 % $NaN_3$ in Wasser, Mittelwert aus drei Zellen.

| Zeit (h) | 0 | 3 | 8,5 | 14 | 19 | 25 | 30 |
|---|---|---|---|---|---|---|---|
| Freisetzung Beispiel 3 | 0,0 | 1,5 | 13,4 | 65,5 | 176,8 | 326,6 | 497,9 |
| Vergleichsbeispiel | 0,0 | 3,2 | 8,6 | 19,3 | 33,0 | 67,1 | 102,0 |

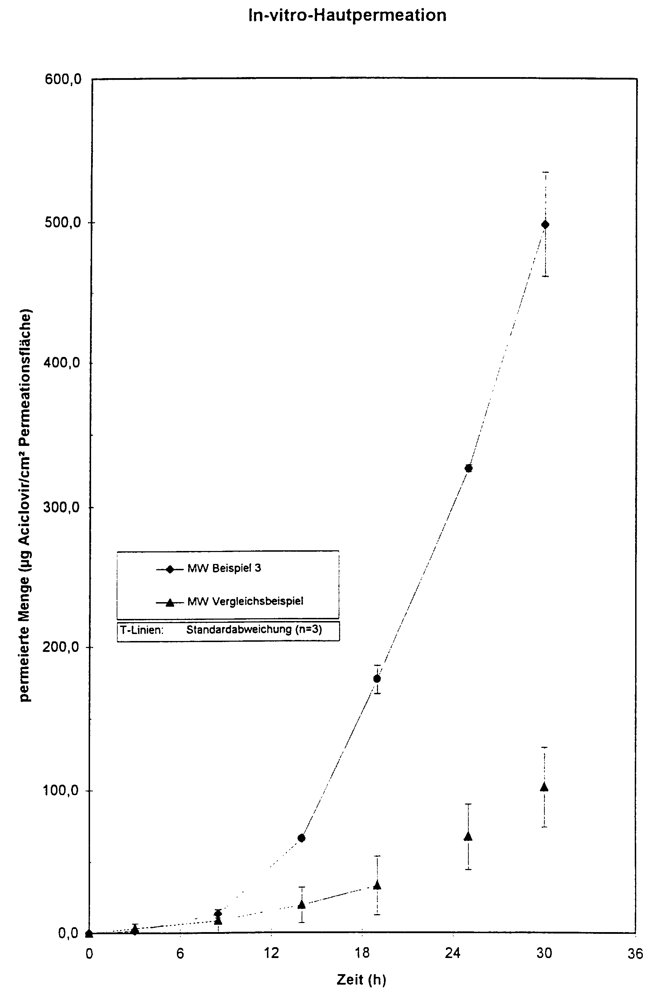

Diese Ergebnisse werden auch noch durch **Figur 1** erläutert.

**Patentansprüche**

1. Wäßrige pharmazeutische Zubereitung zu topischer Applikation mit einem Gehalt an Aciclovir oder an einem Aciclovir-Derivat als Wirkstoff, zusätzlich einer öligen Komponete, einem mehrwertigen Alkohol und einem fakultativen Konsistenzgeber, **gekennzeichnet durch** einen Gehalt an einem Phospolipid, der einer Doppel- oder Mehrschichtstruktur des Phospholipids in der Zubereitung entspricht.

2. Zubereitung nach Anspruch 1, **gekennzeichnet durch** ein gesättigtes oder ungesättigtes Lecithin als Phospholipid.

3. Zubereitung nach Anspruch 1 oder 2, **gekennzeichnet durch** einen Phospholipid-Gehalt von etwa 2 bis etwa 20 und insbesondere bis etwa 10 Gew.-% (bezogen auf die Zubereitung).

4. Zubereitung nach einem der vorhergehenden Ansprüche, **gekennzeichnet durch** einen Wirkstoff-Gehalt von etwa 2 bis etwa 7 Gew.-% (bezogen auf die Zubereitung).

5. Zubereitung nach einem der vorhergehenden Ansprüche, **gekennzeichnet durch** einen Wassergehalt von etwa 15 bis 50 und insbesondere etwa 25 bis etwa 45 Gew.-% (bezogen auf die Zubereitung).

6. Zubereitung nach einem der vorhergehenden Ansprüche, **gekennzeichnet durch** Propylenglycol, Butan-1,3-diol oder Glycerin als mehrwertiger Alkohol.

**EP 0 835 100 B1**

**7.** Zubereitung nach einem der vorhergehenden Ansprüche, **gekennzeichnet durch** einen Gehalt an mehrwertigem Alkohol von etwa 25 bis etwa 45 Gew.-% (bezogen auf die Zubereitung).

**8.** Zubereitung nach einem der vorhergehenden Ansprüche, **gekennzeichnet durch** ein Verhältnis von Wasser zu mehrwertigem Alkohol von etwa 1,5:1 bis etwa 0,75:1.

**9.** Zubereitung nach einem der vorhergehenden Ansprüche, **gekennzeichnet durch** ein Öl auf pflanzlicher Basis und/oder ein mittelkettiges Triglycerid als ölige Komponente.

**10.** Zubereitung nach Anspruch 9, **gekennzeichnet durch** Avocadoöl, Erdnußöl, Sonnenblumenöl, Sesamöl oder Olivenöl als Öl auf pflanzlicher Basis.

**11.** Zubereitung nach einem der vorhergehenden Ansprüche, **gekennzeichnet durch** einen Gehalt an öliger Komponente von etwa 5 bis etwa 40 und insbesondere etwa 10 bis etwa 30 Gew.-% (bezogen auf die Zubereitung).

**12.** Zubereitung nach einem der vorhergehenden Ansprüche, **gekennzeichnet durch** einen Gehalt an $\alpha$-Tocopherol oder $\alpha$-Tocopherol-Derivat, insbesondere von etwa 0,1 bis etwa 5 und insbesondere von etwa 0,5 bis etwa 5 Gew.-% (bezogen auf die Zubereitung).

**13.** Zubereitung nach einem der vorhergehenden Ansprüche, **gekennzeichnet durch** einen Gehalt an Glycerinmonostearat, Cetylstearylalkohol, synthetisches oder natürliches Wachs, Cetylpalmitat, Walrat und/oder Polyacrylsäure, als Konsistenzgeber, insbesondere in einer Menge von etwa 0,5 bis 20 Gew.-%.

**Claims**

**1.** Aqueous pharmaceutical preparation for topical application comprising aciclovir or an aciclovir derivative as active ingredient, and in addition an oily component, a polyhydric alcohol and an optional consistency-providing agent, **characterized by** a content of a phospholipid that corresponds to a double- or multi-layer structure of the phospholipid in the preparation.

**2.** Preparation according to claim 1, **characterized by** an saturated or unsaturated lecithin as phospholipid.

**3.** Preparation according to claim 1 or 2, **characterized by** a phospholipid content of from approximately 2 to approximately 20% by weight and especially up to approximately 10% by weight (based on the preparation).

**4.** Preparation according to any one of the preceding claims, **characterized by** an active ingredient content of from approximately 2 to approximately 7% by weight (based on the preparation).

**5.** Preparation according to any one of the preceding claims, **characterized by** a water content of approximately from 15 to 50% by weight and especially from approximately 25 to approximately 45% by weight (based on the preparation).

**6.** Preparation according to any one of the preceding claims, **characterized by** propylene glycol, butane-1,3-diol or glycerol as polyhydric alcohol.

**7.** Preparation according to any one of the preceding claims, **characterized by** an polyhydric alcohol content of from approximately 25 to approximately 45% by weight (based on the preparation).

**8.** Preparation according to any one of the preceding claims, **characterized by** a ratio of water to polyhydric alcohol of from approximately 2.5:1 to approximately 0.75:1.

**9.** Preparation according to any one of the preceding claims, **characterized by** a plant-based oil and/or a medium-chain triglyceride as oily component.

**10.** Preparation according to claim 9, **characterized by** avocado oil, groundnut oil, sunflower oil, sesame oil or olive oil as plant-based oil.

**11.** Preparation according to any one of the preceding claims, **characterized by** an oily component content of from approximately 5 to approximately 40% by weight, and especially from approximately 10 to approximately 30% by weight (based on the preparation).

**12.** Preparation according to any one of the preceding claims, **characterized by** an $\alpha$-tocopherol or $\alpha$-tocopherol derivative content of especially from approximately 0.1 to approximately 5% by weight, and especially from approximately 0.5 to approximately 5% by weight (based on the preparation).

**13.** Preparation according to any one of the preceding claims, **characterized by** a content of glycerol monostearate, cetyl stearyl alcohol, synthetic or natural wax, cetyl palmitate, spermaceti and/or polyacrylic acid, as consistency-providing agent, especially in an amount of approximately from 0.5 to 20% by weight.


**Revendications**

**1.** Préparation pharmaceutique aqueuse pour application topique contenant de l'Aciclovir ou un dérivé d'Aciclovir comme matière active, en outre, un composant huileux, un alcool polyvalent et un épaississeur facultatif, **caractérisée par** une teneur en un phospholipide qui correspond à une structure à couches doubles ou multiples du phospholipide dans la préparation.

**2.** Préparation selon la revendication 1, **caractérisée par** une lécithine saturée ou insaturée comme phospholipide.

**3.** Préparation selon la revendication 1 ou 2, **caractérisée par** une teneur en phospholipide d'environ 2 à environ 20 et en particulier à environ 10 % en poids (par rapport à la préparation).

**4.** Préparation selon l'une des revendications précédentes, **caractérisée par** une teneur on matière active d'environ 2 à environ 7 % en poids (par rapport à la préparation).

**5.** Préparation selon l'une des revendications précédentes, **caractérisée par** une teneur en eau d'environ 15 à 50 et en particulier d'environ 25 à 45 % en poids (par rapport à la préparation).

**6.** Préparation selon l'une des revendications précédentes, **caractérisée par** du propylène-glycol, du butanediol-1,3 ou de la glycérine comme alcool polyvalent.

**7.** Préparation selon l'une des revendications précédentes, **caractérisée par** une teneur en un alcool polyvalent d'environ 25 à environ 45 % en poids (par rapport à la préparation).

**8.** Préparation selon l'une des revendications précédentes, **caractérisée par** un rapport d'eau à l'alcool polyvalent d'environ 1,5 / l à environ 0,75 / l.

**9.** Préparation selon l'une des revendications précédentes, **caractérisée par** une huile à base végétale et/ou un triglycéride à chaîne moyenne comme composants huileux.

**10.** Préparation selon la revendication 9, **caractérisée par** de l'huile d'avocat, de l'huile de noix, de l'huile de tournesol, de l'huile de sésame ou de l'huile d'olive comme huile à base végétale.

**11.** Préparation selon l'une des revendications précédentes, **caractérisée par** une teneur en composant huileux d'environ 5 à environ 40 et en particulier d'environ 10 à environ 30 % en poids (par rapport à la préparation).

**12.** Préparation selon l'une des revendications précédentes, **caractérisée par** une teneur en $\alpha$-tocophérol ou un dérivé d'$\alpha$-tocophérol, en particulier d'environ 0,1 à environ 5, et en particulier d'environ 0,5 à environ 5 % en poids (par rapport à la préparation).

**13.** Préparation selon l'une des revendications précédentes, **caractérisée par** une teneur en monostéarate de glycérine, en alcool cétylique et alcool stéarique, en cire synthétique ou naturelle, en palmitate cétylique, en blanc de baleine et/ou en acide polyacrylique comme épaississeur, en particulier en une quantité d'environ 0,5 à 20 % en poids.

# In-vitro-Hautpermeation